# EUROPEAN PATENT APPLICATION

(11) **EP 2 606 908 A1**
(43) Date of publication of application: **26.06.2013**
(21) Application number: 11290583.1
(22) Date of filing: 19.12.2011
(51) Int. Cl.: A61K 38/27, A61P 5/00

(54) **Novel pharmaceutical composition for a long acting growth hormone fusion protein (LAGH)**

(71) Applicant: IPSEN PHARMA S.A.S., 91200 Boulogne-Billancourt (FR)
(72) Inventor: Richard, Joël., F-78490 Mere (FR); Baronnet, Marie-Madeleine., F-27530 Ezy Sur Eure (FR); Bertocchi, Laurent., F-27240 Sylvains les Moulins (FR)
(74) Representative: Audonnet, Nathalie

(57) **Abstract**

The object of the present invention is a pharmaceutical liquid composition comprising,
i) a long acting growth hormone fusion protein (LAGH) as active substance ;
ii) a buffer ; and
iii) a salt ;

wherein the pH of the composition is between 5.0 and 7.0.

SEQUENCE LISTING

<110>  Ipsen Pharma S.A.S.

<120>  Novel pharmaceutical composition for a long acting growth hormone
        fusion protein (LAGH)

<130>  IPH 026

<160>  12

<170>  PatentIn version 3.5

<210>  1

<211>  1455

<212>  DNA

<213>  artificial

<220>

<223>  non-natural fusion protein

<400>  1

atggctacag gctcccggac gtccctgctc ctggcttttg gcctgctctg cctgccctgg      60

cttcaagagg gcagtgcctt cccaaccatt cccttatcca ggctttttga caacgctatg     120

ctccgcgccc atcgtctgca ccagctggcc tttgacacct accaggagtt tgaagaagcc     180

tatatcccaa aggaacagaa gtattcattc ctgcagaacc cccagacctc cctctgtttc     240

tcagagtcta ttccgacacc ctccaacagg gaggaaacac aacagaaatc caacctagag     300

ctgctccgca tctccctgct gctcatccag tcgtggctgg agcccgtgca gttcctcagg     360

agtgtcttcg ccaacagcct ggtgtacggc gcctctgaca gcaacgtcta tgacctccta     420

aaggacctag aggaaggcat ccaaacgctg atggggaggc tggaagatgg cagcccccgg     480

actgggcaga tcttcaagca gacctacagc aagttcgaca caaactcaca caacgatgac     540

gcactactca agaactacgg gctgctctac tgcttcagga aggacatgga caaggtcgag     600

acattcctgc gcatcgtgca gtgccgctct gtggagggca gctgtggctt cggcggccgc     660

ggtggcggag gtagtggtgg cggaggtagc ggtggcggag gttctggtgg cggaggttcc     720

gaattctttt ctggaagtga ggccacagca gctatcctta gcagagcacc ctggagtctg     780

caaagtgtta atccaggcct aaagacaaat tcttctaagg agcctaaatt caccaagtgc     840

cgttcacctg agcgagagac tttttcatgc cactggacag atgaggttca tcatggtaca 900

aagaacctag gacccataca gctgttctat accagaagga acactcaaga atggactcaa 960

gaatggaaag aatgccctga ttatgtttct gctggggaaa acagctgtta ctttaattca 1020

tcgtttacct ccatctggat accttattgt atcaagctaa ctagcaatgg tggtacagtg 1080

gatgaaaagt gtttctctgt tgatgaaata gtgcaaccag atccacccat tgccctcaac 1140

tggactttac tgaacgtcag tttaactggg attcatgcag atatccaagt gagatgggaa 1200

gcaccacgca atgcagatat tcagaaagga tggatggttc tggagtatga acttcaatac 1260

aaagaagtaa atgaaactaa atggaaaatg atggacccta tattgacaac atcagttcca 1320

gtgtactcat tgaaagtgga taaggaatat gaagtacgcg tgagatccaa acaacgaaac 1380

tctggaaatt atggcgagtt cagtgaggtg ctctatgtaa cacttcctca gatgagccaa 1440

aagcttttcg aataa 1455

<210> 2

<211> 1443

<212> DNA

<213> artificial

<220>

<223> non-natural fusion protein

<400> 2

atggctacag gctcccggac gtccctgctc ctggcttttg gcctgctctg cctgccctgg 60

cttcaagagg gcagtgcctt cccaaccatt cccttatcca ggctttttga caacgctatg 120

ctccgcgccc atcgtctgca ccagctggcc tttgacacct accaggagtt tgaagaagcc 180

tatatcccaa aggaacagaa gtattcattc ctgcagaacc cccagacctc cctctgtttc 240

tcagagtcta ttccgacacc ctccaacagg gaggaaacac aacagaaatc caacctagag 300

ctgctccgca tctccctgct gctcatccag tcgtggctgg agcccgtgca gttcctcagg 360

agtgtcttcg ccaacagcct ggtgtacggc gcctctgaca gcaacgtcta tgacctccta 420

aaggacctag aggaaggcat ccaaacgctg atggggaggc tggaagatgg cagcccccgg 480

actgggcaga tcttcaagca gacctacagc aagttcgaca caaactcaca caacgatgac 540

gcactactca agaactacgg gctgctctac tgcttcagga aggacatgga caaggtcgag 600

acattcctgc gcatcgtgca gtgccgctct gtggagggca gctgtggctt cggcggccgc 660

ggtggcggag gtagtggtgg cggaggtagc ggtggcggag gttctggtgg cggaggttcc 720

gaattctttt ctggaagtga ggccacagca gctatcctta gcagagcacc ctggagtctg 780

caaagtgtta atccaggcct aaagacaaat tcttctaagg agcctaaatt caccaagtgc 840

cgttcacctg agcgagagac tttttcatgc cactggacag atgaggttca tcatggtaca 900

aagaacctag gacccataca gctgttctat accagaagga acactcaaga atggactcaa 960

gaatggaaag aatgccctga ttatgtttct gctggggaaa acagctgtta ctttaattca 1020

tcgtttacct ccatctggat accttattgt atcaagctaa ctagcaatgg tggtacagtg 1080

gatgaaaagt gtttctctgt tgatgaaata gtgcaaccag atccacccat tgccctcaac 1140

tggactttac tgaacgtcag tttaactggg attcatgcag atatccaagt gagatgggaa 1200

gcaccacgca atgcagatat tcagaaagga tggatggttc tggagtatga acttcaatac 1260

aaagaagtaa atgaaactaa atggaaaatg atggacccta tattgacaac atcagttcca 1320

gtgtactcat tgaaagtgga taaggaatat gaagtgcgtg tgagatccaa acaacgaaac 1380

tctggaaatt atggcgagtt cagtgaggtg ctctatgtaa cacttcctca gatgagccaa 1440

taa 1443

<210> 3

<211> 1443

<212> DNA

<213> artificial

<220>

<223> non-natural fusion protein

<400> 3

atggctacag gctcccggac gtccctgctc ctggcttttg gcctgctctg cctgccctgg 60

cttcaagagg gcagtgcctt cccaaccatt cccttatcca ggctttttga caacgctatg 120

ctccgcgccc atcgtctgca ccagctggcc tttgacacct accaggagtt tgaagaagcc 180

tatatcccaa aggaacagaa gtattcattc ctgcagaacc cccagacctc cctctgtttc 240

tcagagtcta ttccgacacc ctccaacagg gaggaaacac aacagaaatc caacctagag 300

ctgctccgca tctccctgct gctcatccag tcgtggctgg agcccgtgca gttcctcagg 360

agtgtcttcg ccaacagcct ggtgtacggc gcctctgaca gcaacgtcta tgacctccta 420

aaggacctag aggaaggcat ccaaacgctg atggggaggc tggaagatgg cagcccccgg 480

actgggcaga tcttcaagca gacctacagc aagttcgaca caaactcaca caacgatgac 540

gcactactca agaactacgg gctgctctac tgcttcagga aggacatgga caaggtcgag 600

acattcctgc gcatcgtgca gtgccgctct gtggagggca gctgtggctt cggtggcgga 660

ggtagtggtg gcggaggtag cggtggcgga ggttctggtg gcggaggttc cggtggcgga 720

ggtagttttt ctggaagtga ggccacagca gctatcctta gcagagcacc ctggagtctg 780

caaagtgtta atccaggcct aaagacaaat tcttctaagg agcctaaatt caccaagtgc 840

cgttcacctg agcgagagac tttttcatgc cactggacag atgaggttca tcatggtaca 900

aagaacctag gacccataca gctgttctat accagaagga acactcaaga atggactcaa 960

gaatggaaag aatgccctga ttatgtttct gctggggaaa acagctgtta ctttaattca 1020

tcgtttacct ccatctggat accttattgt atcaagctaa ctagcaatgg tggtacagtg 1080

gatgaaaagt gtttctctgt tgatgaaata gtgcaaccag atccacccat tgccctcaac 1140

tggactttac tgaacgtcag tttaactggg attcatgcag atatccaagt gagatgggaa 1200

gcaccacgca atgcagatat tcagaaagga tggatggttc tggagtatga acttcaatac 1260

aaagaagtaa atgaaactaa atggaaaatg atggacccta tattgacaac atcagttcca 1320

gtgtactcat tgaaagtgga taaggaatat gaagtgcgtg tgagatccaa acaacgaaac 1380

tctggaaatt atggcgagtt cagtgaggtg ctctatgtaa cacttcctca gatgagccaa 1440

taa 1443

<210> 4

<211> 1368

<212> DNA

<213> artificial

<220>

<223> non-natural fusion protein

<400> 4

atggctacag gctcccggac gtccctgctc ctggcttttg gcctgctctg cctgccctgg 60

cttcaagagg gcagtgcctt cccaaccatt cccttatcca ggctttttga caacgctatg 120

ctccgcgccc atcgtctgca ccagctggcc tttgacacct accaggagtt tgaagaagcc 180

tatatcccaa aggaacagaa gtattcattc ctgcagaacc cccagacctc cctctgtttc 240

tcagagtcta ttccgacacc ctccaacagg gaggaaacac aacagaaatc caacctagag 300

ctgctccgca tctccctgct gctcatccag tcgtggctgg agcccgtgca gttcctcagg 360

agtgtcttcg ccaacagcct ggtgtacggc gcctctgaca gcaacgtcta tgacctccta 420

aaggacctag aggaaggcat ccaaacgctg atggggaggc tggaagatgg cagcccccgg 480

actgggcaga tcttcaagca gacctacagc aagttcgaca caaactcaca caacgatgac 540

gcactactca agaactacgg gctgctctac tgcttcagga aggacatgga caaggtcgag 600

acattcctgc gcatcgtgca gtgccgctct gtggagggca gctgtggctt cttttctgga 660

agtgaggcca cagcagctat ccttagcaga gcaccctgga gtctgcaaag tgttaatcca 720

ggcctaaaga caaattcttc taaggagcct aaattcacca agtgccgttc acctgagcga 780

gagacttttt catgccactg gacagatgag gttcatcatg gtacaaagaa cctaggaccc 840

atacagctgt tctataccag aaggaacact caagaatgga ctcaagaatg gaaagaatgc 900

cctgattatg tttctgctgg ggaaaacagc tgttacttta attcatcgtt tacctccatc 960

tggatacctt attgtatcaa gctaactagc aatggtggta cagtggatga aaagtgtttc 1020

tctgttgatg aaatagtgca accagatcca cccattgccc tcaactggac tttactgaac 1080

gtcagtttaa ctgggattca tgcagatatc caagtgagat gggaagcacc acgcaatgca 1140

gatattcaga aaggatggat ggttctggag tatgaacttc aatacaaaga agtaaatgaa 1200

actaaatgga aaatgatgga ccctatattg acaacatcag ttccagtgta ctcattgaaa 1260

gtggataagg aatatgaagt gcgtgtgaga tccaaacaac gaaactctgg aaattatggc 1320

gagttcagtg aggtgctcta tgtaacactt cctcagatga gccaataa 1368

<210> 5

<211> 484

<212> PRT

<213> artificial

<220>

<223> non-natural fusion protein

<400> 5

Met Ala Thr Gly Ser Arg Thr Ser Leu Leu Leu Ala Phe Gly Leu Leu

1               5                   10                  15

Cys Leu Pro Trp Leu Gln Glu Gly Ser Ala Phe Pro Thr Ile Pro Leu

            20                  25                  30

Ser Arg Leu Phe Asp Asn Ala Met Leu Arg Ala His Arg Leu His Gln

        35                  40                  45

Leu Ala Phe Asp Thr Tyr Gln Glu Phe Glu Glu Ala Tyr Ile Pro Lys

    50                  55                  60

Glu Gln Lys Tyr Ser Phe Leu Gln Asn Pro Gln Thr Ser Leu Cys Phe
65                  70                  75                  80
Ser Glu Ser Ile Pro Thr Pro Ser Asn Arg Glu Glu Thr Gln Gln Lys
                85                  90                  95
Ser Asn Leu Glu Leu Leu Arg Ile Ser Leu Leu Leu Ile Gln Ser Trp
            100                 105                 110
Leu Glu Pro Val Gln Phe Leu Arg Ser Val Phe Ala Asn Ser Leu Val
        115                 120                 125
Tyr Gly Ala Ser Asp Ser Asn Val Tyr Asp Leu Leu Lys Asp Leu Glu
    130                 135                 140
Glu Gly Ile Gln Thr Leu Met Gly Arg Leu Glu Asp Gly Ser Pro Arg
145                 150                 155                 160
Thr Gly Gln Ile Phe Lys Gln Thr Tyr Ser Lys Phe Asp Thr Asn Ser
                165                 170                 175
His Asn Asp Asp Ala Leu Leu Lys Asn Tyr Gly Leu Leu Tyr Cys Phe
            180                 185                 190
Arg Lys Asp Met Asp Lys Val Glu Thr Phe Leu Arg Ile Val Gln Cys
        195                 200                 205
Arg Ser Val Glu Gly Ser Cys Gly Phe Gly Gly Arg Gly Gly Gly Gly
    210                 215                 220
Ser Gly Gly Gly Gly Ser Gly Gly Gly Gly Ser Gly Gly Gly Gly Ser
225                 230                 235                 240
Glu Phe Phe Ser Gly Ser Glu Ala Thr Ala Ala Ile Leu Ser Arg Ala
                245                 250                 255
Pro Trp Ser Leu Gln Ser Val Asn Pro Gly Leu Lys Thr Asn Ser Ser
            260                 265                 270
Lys Glu Pro Lys Phe Thr Lys Cys Arg Ser Pro Glu Arg Glu Thr Phe
        275                 280                 285
Ser Cys His Trp Thr Asp Glu Val His His Gly Thr Lys Asn Leu Gly
    290                 295                 300
Pro Ile Gln Leu Phe Tyr Thr Arg Arg Asn Thr Gln Glu Trp Thr Gln

305                 310                 315                 320

Glu Trp Lys Glu Cys Pro Asp Tyr Val Ser Ala Gly Glu Asn Ser Cys

                325                 330                 335

Tyr Phe Asn Ser Ser Phe Thr Ser Ile Trp Ile Pro Tyr Cys Ile Lys

            340                 345                 350

Leu Thr Ser Asn Gly Gly Thr Val Asp Glu Lys Cys Phe Ser Val Asp

        355                 360                 365

Glu Ile Val Gln Pro Asp Pro Pro Ile Ala Leu Asn Trp Thr Leu Leu

    370                 375                 380

Asn Val Ser Leu Thr Gly Ile His Ala Asp Ile Gln Val Arg Trp Glu

385                 390                 395                 400

Ala Pro Arg Asn Ala Asp Ile Gln Lys Gly Trp Met Val Leu Glu Tyr

                405                 410                 415

Glu Leu Gln Tyr Lys Glu Val Asn Glu Thr Lys Trp Lys Met Met Asp

            420                 425                 430

Pro Ile Leu Thr Thr Ser Val Pro Val Tyr Ser Leu Lys Val Asp Lys

        435                 440                 445

Glu Tyr Glu Val Arg Val Arg Ser Lys Gln Arg Asn Ser Gly Asn Tyr

    450                 455                 460

Gly Glu Phe Ser Glu Val Leu Tyr Val Thr Leu Pro Gln Met Ser Gln

465                 470                 475                 480

Lys Leu Phe Glu

<210>  6

<211>  458

<212>  PRT

<213>  artificial

<220>

<223>  non-natural fusion protein

<400>  6

Phe Pro Thr Ile Pro Leu Ser Arg Leu Phe Asp Asn Ala Met Leu Arg

1               5                   10                  15

Ala His Arg Leu His Gln Leu Ala Phe Asp Thr Tyr Gln Glu Phe Glu

            20                  25                  30

Glu Ala Tyr Ile Pro Lys Glu Gln Lys Tyr Ser Phe Leu Gln Asn Pro

        35                  40                  45

Gln Thr Ser Leu Cys Phe Ser Glu Ser Ile Pro Thr Pro Ser Asn Arg

    50                  55                  60

Glu Glu Thr Gln Gln Lys Ser Asn Leu Glu Leu Leu Arg Ile Ser Leu

65                  70                  75                  80

Leu Leu Ile Gln Ser Trp Leu Glu Pro Val Gln Phe Leu Arg Ser Val

                85                  90                  95

Phe Ala Asn Ser Leu Val Tyr Gly Ala Ser Asp Ser Asn Val Tyr Asp

            100                 105                 110

Leu Leu Lys Asp Leu Glu Glu Gly Ile Gln Thr Leu Met Gly Arg Leu

        115                 120                 125

Glu Asp Gly Ser Pro Arg Thr Gly Gln Ile Phe Lys Gln Thr Tyr Ser

    130                 135                 140

Lys Phe Asp Thr Asn Ser His Asn Asp Asp Ala Leu Leu Lys Asn Tyr

145                 150                 155                 160

Gly Leu Leu Tyr Cys Phe Arg Lys Asp Met Asp Lys Val Glu Thr Phe

                165                 170                 175

Leu Arg Ile Val Gln Cys Arg Ser Val Glu Gly Ser Cys Gly Phe Gly

            180                 185                 190

Gly Arg Gly Gly Gly Gly Ser Gly Gly Gly Gly Ser Gly Gly Gly Gly

        195                 200                 205

Ser Gly Gly Gly Gly Ser Glu Phe Phe Ser Gly Ser Glu Ala Thr Ala

    210                 215                 220

Ala Ile Leu Ser Arg Ala Pro Trp Ser Leu Gln Ser Val Asn Pro Gly

225                 230                 235                 240

Leu Lys Thr Asn Ser Ser Lys Glu Pro Lys Phe Thr Lys Cys Arg Ser

                245                 250                 255

Pro Glu Arg Glu Thr Phe Ser Cys His Trp Thr Asp Glu Val His His
            260                 265                 270
Gly Thr Lys Asn Leu Gly Pro Ile Gln Leu Phe Tyr Thr Arg Arg Asn
        275                 280                 285
Thr Gln Glu Trp Thr Gln Glu Trp Lys Glu Cys Pro Asp Tyr Val Ser
    290                 295                 300
Ala Gly Glu Asn Ser Cys Tyr Phe Asn Ser Ser Phe Thr Ser Ile Trp
305                 310                 315                 320
Ile Pro Tyr Cys Ile Lys Leu Thr Ser Asn Gly Gly Thr Val Asp Glu
                325                 330                 335
Lys Cys Phe Ser Val Asp Glu Ile Val Gln Pro Asp Pro Pro Ile Ala
            340                 345                 350
Leu Asn Trp Thr Leu Leu Asn Val Ser Leu Thr Gly Ile His Ala Asp
        355                 360                 365
Ile Gln Val Arg Trp Glu Ala Pro Arg Asn Ala Asp Ile Gln Lys Gly
    370                 375                 380
Trp Met Val Leu Glu Tyr Glu Leu Gln Tyr Lys Glu Val Asn Glu Thr
385                 390                 395                 400
Lys Trp Lys Met Met Asp Pro Ile Leu Thr Thr Ser Val Pro Val Tyr
                405                 410                 415
Ser Leu Lys Val Asp Lys Glu Tyr Glu Val Arg Val Arg Ser Lys Gln
            420                 425                 430
Arg Asn Ser Gly Asn Tyr Gly Glu Phe Ser Glu Val Leu Tyr Val Thr
        435                 440                 445
Leu Pro Gln Met Ser Gln Lys Leu Phe Glu
    450                 455

<210> 7
<211> 480
<212> PRT
<213> artificial

<220>

<223>  non-natural fusion protein

<400>  7

Met Ala Thr Gly Ser Arg Thr Ser Leu Leu Leu Ala Phe Gly Leu Leu
1               5                   10                  15

Cys Leu Pro Trp Leu Gln Glu Gly Ser Ala Phe Pro Thr Ile Pro Leu
            20                  25                  30

Ser Arg Leu Phe Asp Asn Ala Met Leu Arg Ala His Arg Leu His Gln
        35                  40                  45

Leu Ala Phe Asp Thr Tyr Gln Glu Phe Glu Glu Ala Tyr Ile Pro Lys
    50                  55                  60

Glu Gln Lys Tyr Ser Phe Leu Gln Asn Pro Gln Thr Ser Leu Cys Phe
65                  70                  75                  80

Ser Glu Ser Ile Pro Thr Pro Ser Asn Arg Glu Glu Thr Gln Gln Lys
                85                  90                  95

Ser Asn Leu Glu Leu Leu Arg Ile Ser Leu Leu Leu Ile Gln Ser Trp
            100                 105                 110

Leu Glu Pro Val Gln Phe Leu Arg Ser Val Phe Ala Asn Ser Leu Val
        115                 120                 125

Tyr Gly Ala Ser Asp Ser Asn Val Tyr Asp Leu Leu Lys Asp Leu Glu
    130                 135                 140

Glu Gly Ile Gln Thr Leu Met Gly Arg Leu Glu Asp Gly Ser Pro Arg
145                 150                 155                 160

Thr Gly Gln Ile Phe Lys Gln Thr Tyr Ser Lys Phe Asp Thr Asn Ser
                165                 170                 175

His Asn Asp Asp Ala Leu Leu Lys Asn Tyr Gly Leu Leu Tyr Cys Phe
            180                 185                 190

Arg Lys Asp Met Asp Lys Val Glu Thr Phe Leu Arg Ile Val Gln Cys
        195                 200                 205

Arg Ser Val Glu Gly Ser Cys Gly Phe Gly Gly Arg Gly Gly Gly Gly
    210                 215                 220

Ser Gly Gly Gly Gly Ser Gly Gly Gly Gly Ser Gly Gly Gly Gly Ser
225                 230                 235                 240
Glu Phe Phe Ser Gly Ser Glu Ala Thr Ala Ala Ile Leu Ser Arg Ala
                245                 250                 255
Pro Trp Ser Leu Gln Ser Val Asn Pro Gly Leu Lys Thr Asn Ser Ser
            260                 265                 270
Lys Glu Pro Lys Phe Thr Lys Cys Arg Ser Pro Glu Arg Glu Thr Phe
        275                 280                 285
Ser Cys His Trp Thr Asp Glu Val His His Gly Thr Lys Asn Leu Gly
    290                 295                 300
Pro Ile Gln Leu Phe Tyr Thr Arg Arg Asn Thr Gln Glu Trp Thr Gln
305                 310                 315                 320
Glu Trp Lys Glu Cys Pro Asp Tyr Val Ser Ala Gly Glu Asn Ser Cys
                325                 330                 335
Tyr Phe Asn Ser Ser Phe Thr Ser Ile Trp Ile Pro Tyr Cys Ile Lys
            340                 345                 350
Leu Thr Ser Asn Gly Gly Thr Val Asp Glu Lys Cys Phe Ser Val Asp
        355                 360                 365
Glu Ile Val Gln Pro Asp Pro Pro Ile Ala Leu Asn Trp Thr Leu Leu
    370                 375                 380
Asn Val Ser Leu Thr Gly Ile His Ala Asp Ile Gln Val Arg Trp Glu
385                 390                 395                 400
Ala Pro Arg Asn Ala Asp Ile Gln Lys Gly Trp Met Val Leu Glu Tyr
                405                 410                 415
Glu Leu Gln Tyr Lys Glu Val Asn Glu Thr Lys Trp Lys Met Met Asp
            420                 425                 430
Pro Ile Leu Thr Thr Ser Val Pro Val Tyr Ser Leu Lys Val Asp Lys
        435                 440                 445
Glu Tyr Glu Val Arg Val Arg Ser Lys Gln Arg Asn Ser Gly Asn Tyr
    450                 455                 460
Gly Glu Phe Ser Glu Val Leu Tyr Val Thr Leu Pro Gln Met Ser Gln

465                 470                 475                 480

<210>  8

<211>  454

<212>  PRT

<213>  artificial

<220>

<223>  non-natural fusion protein

<400>  8

Phe Pro Thr Ile Pro Leu Ser Arg Leu Phe Asp Asn Ala Met Leu Arg

1               5                   10                  15

Ala His Arg Leu His Gln Leu Ala Phe Asp Thr Tyr Gln Glu Phe Glu

            20                  25                  30

Glu Ala Tyr Ile Pro Lys Glu Gln Lys Tyr Ser Phe Leu Gln Asn Pro

        35                  40                  45

Gln Thr Ser Leu Cys Phe Ser Glu Ser Ile Pro Thr Pro Ser Asn Arg

    50                  55                  60

Glu Glu Thr Gln Gln Lys Ser Asn Leu Glu Leu Leu Arg Ile Ser Leu

65                  70                  75                  80

Leu Leu Ile Gln Ser Trp Leu Glu Pro Val Gln Phe Leu Arg Ser Val

                85                  90                  95

Phe Ala Asn Ser Leu Val Tyr Gly Ala Ser Asp Ser Asn Val Tyr Asp

            100                 105                 110

Leu Leu Lys Asp Leu Glu Glu Gly Ile Gln Thr Leu Met Gly Arg Leu

        115                 120                 125

Glu Asp Gly Ser Pro Arg Thr Gly Gln Ile Phe Lys Gln Thr Tyr Ser

    130                 135                 140

Lys Phe Asp Thr Asn Ser His Asn Asp Asp Ala Leu Leu Lys Asn Tyr

145                 150                 155                 160

Gly Leu Leu Tyr Cys Phe Arg Lys Asp Met Asp Lys Val Glu Thr Phe

                165                 170                 175

Leu Arg Ile Val Gln Cys Arg Ser Val Glu Gly Ser Cys Gly Phe Gly
            180                 185                 190
Gly Arg Gly Gly Gly Gly Ser Gly Gly Gly Gly Ser Gly Gly Gly Gly
        195                 200                 205
Ser Gly Gly Gly Gly Ser Glu Phe Phe Ser Gly Ser Glu Ala Thr Ala
    210                 215                 220
Ala Ile Leu Ser Arg Ala Pro Trp Ser Leu Gln Ser Val Asn Pro Gly
225                 230                 235                 240
Leu Lys Thr Asn Ser Ser Lys Glu Pro Lys Phe Thr Lys Cys Arg Ser
                245                 250                 255
Pro Glu Arg Glu Thr Phe Ser Cys His Trp Thr Asp Glu Val His His
            260                 265                 270
Gly Thr Lys Asn Leu Gly Pro Ile Gln Leu Phe Tyr Thr Arg Arg Asn
        275                 280                 285
Thr Gln Glu Trp Thr Gln Glu Trp Lys Glu Cys Pro Asp Tyr Val Ser
    290                 295                 300
Ala Gly Glu Asn Ser Cys Tyr Phe Asn Ser Ser Phe Thr Ser Ile Trp
305                 310                 315                 320
Ile Pro Tyr Cys Ile Lys Leu Thr Ser Asn Gly Gly Thr Val Asp Glu
                325                 330                 335
Lys Cys Phe Ser Val Asp Glu Ile Val Gln Pro Asp Pro Pro Ile Ala
            340                 345                 350
Leu Asn Trp Thr Leu Leu Asn Val Ser Leu Thr Gly Ile His Ala Asp
        355                 360                 365
Ile Gln Val Arg Trp Glu Ala Pro Arg Asn Ala Asp Ile Gln Lys Gly
    370                 375                 380
Trp Met Val Leu Glu Tyr Glu Leu Gln Tyr Lys Glu Val Asn Glu Thr
385                 390                 395                 400
Lys Trp Lys Met Met Asp Pro Ile Leu Thr Thr Ser Val Pro Val Tyr
                405                 410                 415
Ser Leu Lys Val Asp Lys Glu Tyr Glu Val Arg Val Arg Ser Lys Gln

420                 425                 430

Arg Asn Ser Gly Asn Tyr Gly Glu Phe Ser Glu Val Leu Tyr Val Thr

        435                 440                 445

Leu Pro Gln Met Ser Gln

    450

<210>  9

<211>  480

<212>  PRT

<213>  artificial

<220>

<223>  non-natural fusion protein

<400>  9
   Met Ala Thr Gly Ser Arg Thr Ser Leu Leu Leu Ala Phe Gly Leu Leu
   1               5                   10                  15
   Cys Leu Pro Trp Leu Gln Glu Gly Ser Ala Phe Pro Thr Ile Pro Leu
               20                  25                  30
   Ser Arg Leu Phe Asp Asn Ala Met Leu Arg Ala His Arg Leu His Gln
           35                  40                  45
   Leu Ala Phe Asp Thr Tyr Gln Glu Phe Glu Glu Ala Tyr Ile Pro Lys
       50                  55                  60
   Glu Gln Lys Tyr Ser Phe Leu Gln Asn Pro Gln Thr Ser Leu Cys Phe
   65                  70                  75                  80
   Ser Glu Ser Ile Pro Thr Pro Ser Asn Arg Glu Glu Thr Gln Gln Lys
                   85                  90                  95
   Ser Asn Leu Glu Leu Leu Arg Ile Ser Leu Leu Leu Ile Gln Ser Trp
               100                 105                 110
   Leu Glu Pro Val Gln Phe Leu Arg Ser Val Phe Ala Asn Ser Leu Val
           115                 120                 125
   Tyr Gly Ala Ser Asp Ser Asn Val Tyr Asp Leu Leu Lys Asp Leu Glu
       130                 135                 140
   Glu Gly Ile Gln Thr Leu Met Gly Arg Leu Glu Asp Gly Ser Pro Arg
   145                 150                 155                 160
   Thr Gly Gln Ile Phe Lys Gln Thr Tyr Ser Lys Phe Asp Thr Asn Ser
                   165                 170                 175
   His Asn Asp Asp Ala Leu Leu Lys Asn Tyr Gly Leu Leu Tyr Cys Phe
               180                 185                 190
   Arg Lys Asp Met Asp Lys Val Glu Thr Phe Leu Arg Ile Val Gln Cys
           195                 200                 205
   Arg Ser Val Glu Gly Ser Cys Gly Phe Gly Gly Gly Gly Ser Gly Gly
       210                 215                 220
   Gly Gly Ser Gly Gly Gly Gly Ser Gly Gly Gly Gly Ser Gly Gly Gly
   225                 230                 235                 240
   Gly Ser Phe Ser Gly Ser Glu Ala Thr Ala Ala Ile Leu Ser Arg Ala
                   245                 250                 255
   Pro Trp Ser Leu Gln Ser Val Asn Pro Gly Leu Lys Thr Asn Ser Ser
               260                 265                 270
   Lys Glu Pro Lys Phe Thr Lys Cys Arg Ser Pro Glu Arg Glu Thr Phe
           275                 280                 285
   Ser Cys His Trp Thr Asp Glu Val His His Gly Thr Lys Asn Leu Gly
       290                 295                 300
   Pro Ile Gln Leu Phe Tyr Thr Arg Arg Asn Thr Gln Glu Trp Thr Gln
   305                 310                 315                 320
   Glu Trp Lys Glu Cys Pro Asp Tyr Val Ser Ala Gly Glu Asn Ser Cys
                   325                 330                 335
   Tyr Phe Asn Ser Ser Phe Thr Ser Ile Trp Ile Pro Tyr Cys Ile Lys
               340                 345                 350
   Leu Thr Ser Asn Gly Gly Thr Val Asp Glu Lys Cys Phe Ser Val Asp
           355                 360                 365
   Glu Ile Val Gln Pro Asp Pro Pro Ile Ala Leu Asn Trp Thr Leu Leu
       370                 375                 380
   Asn Val Ser Leu Thr Gly Ile His Ala Asp Ile Gln Val Arg Trp Glu
   385                 390                 395                 400
   Ala Pro Arg Asn Ala Asp Ile Gln Lys Gly Trp Met Val Leu Glu Tyr
                   405                 410                 415
   Glu Leu Gln Tyr Lys Glu Val Asn Glu Thr Lys Trp Lys Met Met Asp
               420                 425                 430
   Pro Ile Leu Thr Thr Ser Val Pro Val Tyr Ser Leu Lys Val Asp Lys
           435                 440                 445
   Glu Tyr Glu Val Arg Val Arg Ser Lys Gln Arg Asn Ser Gly Asn Tyr
       450                 455                 460
   Gly Glu Phe Ser Glu Val Leu Tyr Val Thr Leu Pro Gln Met Ser Gln
   465                 470                 475                 480

<210>  10

<211>  454

<212>  PRT

<213>  artificial

<220>

<223>  non-natural fusion protein

<400>  10

Phe Pro Thr Ile Pro Leu Ser Arg Leu Phe Asp Asn Ala Met Leu Arg

1               5                   10                  15

Ala His Arg Leu His Gln Leu Ala Phe Asp Thr Tyr Gln Glu Phe Glu

            20                  25                  30

Glu Ala Tyr Ile Pro Lys Glu Gln Lys Tyr Ser Phe Leu Gln Asn Pro

        35                  40                  45

Gln Thr Ser Leu Cys Phe Ser Glu Ser Ile Pro Thr Pro Ser Asn Arg

    50                  55                  60

Glu Glu Thr Gln Gln Lys Ser Asn Leu Glu Leu Leu Arg Ile Ser Leu

65                  70                  75                  80

Leu Leu Ile Gln Ser Trp Leu Glu Pro Val Gln Phe Leu Arg Ser Val

                85                  90                  95
Phe Ala Asn Ser Leu Val Tyr Gly Ala Ser Asp Ser Asn Val Tyr Asp
            100                 105                 110
Leu Leu Lys Asp Leu Glu Glu Gly Ile Gln Thr Leu Met Gly Arg Leu
        115                 120                 125
Glu Asp Gly Ser Pro Arg Thr Gly Gln Ile Phe Lys Gln Thr Tyr Ser
    130                 135                 140
Lys Phe Asp Thr Asn Ser His Asn Asp Asp Ala Leu Leu Lys Asn Tyr
145                 150                 155                 160
Gly Leu Leu Tyr Cys Phe Arg Lys Asp Met Asp Lys Val Glu Thr Phe
                165                 170                 175
Leu Arg Ile Val Gln Cys Arg Ser Val Glu Gly Ser Cys Gly Phe Gly
            180                 185                 190
Gly Gly Gly Ser Gly Gly Gly Gly Ser Gly Gly Gly Gly Ser Gly Gly
        195                 200                 205
Gly Gly Ser Gly Gly Gly Gly Ser Phe Ser Gly Ser Glu Ala Thr Ala
    210                 215                 220
Ala Ile Leu Ser Arg Ala Pro Trp Ser Leu Gln Ser Val Asn Pro Gly
225                 230                 235                 240
Leu Lys Thr Asn Ser Ser Lys Glu Pro Lys Phe Thr Lys Cys Arg Ser
                245                 250                 255
Pro Glu Arg Glu Thr Phe Ser Cys His Trp Thr Asp Glu Val His His
            260                 265                 270
Gly Thr Lys Asn Leu Gly Pro Ile Gln Leu Phe Tyr Thr Arg Arg Asn
        275                 280                 285
Thr Gln Glu Trp Thr Gln Glu Trp Lys Glu Cys Pro Asp Tyr Val Ser
    290                 295                 300
Ala Gly Glu Asn Ser Cys Tyr Phe Asn Ser Ser Phe Thr Ser Ile Trp
305                 310                 315                 320
Ile Pro Tyr Cys Ile Lys Leu Thr Ser Asn Gly Gly Thr Val Asp Glu
                325                 330                 335

Lys Cys Phe Ser Val Asp Glu Ile Val Gln Pro Asp Pro Pro Ile Ala

            340                 345                 350

Leu Asn Trp Thr Leu Leu Asn Val Ser Leu Thr Gly Ile His Ala Asp

        355                 360                 365

Ile Gln Val Arg Trp Glu Ala Pro Arg Asn Ala Asp Ile Gln Lys Gly

    370                 375                 380

Trp Met Val Leu Glu Tyr Glu Leu Gln Tyr Lys Glu Val Asn Glu Thr

385                 390                 395                 400

Lys Trp Lys Met Met Asp Pro Ile Leu Thr Thr Ser Val Pro Val Tyr

                405                 410                 415

Ser Leu Lys Val Asp Lys Glu Tyr Glu Val Arg Val Arg Ser Lys Gln

            420                 425                 430

Arg Asn Ser Gly Asn Tyr Gly Glu Phe Ser Glu Val Leu Tyr Val Thr

        435                 440                 445

Leu Pro Gln Met Ser Gln

    450

<210> 11

<211> 455

<212> PRT

<213> artificial

<220>

<223> non-natural fusion protein

<400> 11

Met Ala Thr Gly Ser Arg Thr Ser Leu Leu Leu Ala Phe Gly Leu Leu

1               5                   10                  15

Cys Leu Pro Trp Leu Gln Glu Gly Ser Ala Phe Pro Thr Ile Pro Leu

            20                  25                  30

Ser Arg Leu Phe Asp Asn Ala Met Leu Arg Ala His Arg Leu His Gln

        35                  40                  45

Leu Ala Phe Asp Thr Tyr Gln Glu Phe Glu Glu Ala Tyr Ile Pro Lys

50                  55                  60

Glu Gln Lys Tyr Ser Phe Leu Gln Asn Pro Gln Thr Ser Leu Cys Phe

65                  70                  75                  80

Ser Glu Ser Ile Pro Thr Pro Ser Asn Arg Glu Glu Thr Gln Gln Lys

                85                  90                  95

Ser Asn Leu Glu Leu Leu Arg Ile Ser Leu Leu Leu Ile Gln Ser Trp

            100                 105                 110

Leu Glu Pro Val Gln Phe Leu Arg Ser Val Phe Ala Asn Ser Leu Val

        115                 120                 125

Tyr Gly Ala Ser Asp Ser Asn Val Tyr Asp Leu Leu Lys Asp Leu Glu

    130                 135                 140

Glu Gly Ile Gln Thr Leu Met Gly Arg Leu Glu Asp Gly Ser Pro Arg

145                 150                 155                 160

Thr Gly Gln Ile Phe Lys Gln Thr Tyr Ser Lys Phe Asp Thr Asn Ser

                165                 170                 175

His Asn Asp Asp Ala Leu Leu Lys Asn Tyr Gly Leu Leu Tyr Cys Phe

            180                 185                 190

Arg Lys Asp Met Asp Lys Val Glu Thr Phe Leu Arg Ile Val Gln Cys

        195                 200                 205

Arg Ser Val Glu Gly Ser Cys Gly Phe Phe Ser Gly Ser Glu Ala Thr

    210                 215                 220

Ala Ala Ile Leu Ser Arg Ala Pro Trp Ser Leu Gln Ser Val Asn Pro

225                 230                 235                 240

Gly Leu Lys Thr Asn Ser Ser Lys Glu Pro Lys Phe Thr Lys Cys Arg

                245                 250                 255

Ser Pro Glu Arg Glu Thr Phe Ser Cys His Trp Thr Asp Glu Val His

            260                 265                 270

His Gly Thr Lys Asn Leu Gly Pro Ile Gln Leu Phe Tyr Thr Arg Arg

        275                 280                 285

Asn Thr Gln Glu Trp Thr Gln Glu Trp Lys Glu Cys Pro Asp Tyr Val

    290                 295                 300

Ser Ala Gly Glu Asn Ser Cys Tyr Phe Asn Ser Ser Phe Thr Ser Ile

305                 310                 315                 320

Trp Ile Pro Tyr Cys Ile Lys Leu Thr Ser Asn Gly Gly Thr Val Asp

                325                 330                 335

Glu Lys Cys Phe Ser Val Asp Glu Ile Val Gln Pro Asp Pro Pro Ile

            340                 345                 350

Ala Leu Asn Trp Thr Leu Leu Asn Val Ser Leu Thr Gly Ile His Ala

        355                 360                 365

Asp Ile Gln Val Arg Trp Glu Ala Pro Arg Asn Ala Asp Ile Gln Lys

    370                 375                 380

Gly Trp Met Val Leu Glu Tyr Glu Leu Gln Tyr Lys Glu Val Asn Glu

385                 390                 395                 400

Thr Lys Trp Lys Met Met Asp Pro Ile Leu Thr Thr Ser Val Pro Val

                405                 410                 415

Tyr Ser Leu Lys Val Asp Lys Glu Tyr Glu Val Arg Val Arg Ser Lys

            420                 425                 430

Gln Arg Asn Ser Gly Asn Tyr Gly Glu Phe Ser Glu Val Leu Tyr Val

        435                 440                 445

Thr Leu Pro Gln Met Ser Gln

    450                 455

<210>  12

<211>  429

<212>  PRT

<213>  artificial

<220>

<223>  non-natural fusion protein

<400>  12

Phe Pro Thr Ile Pro Leu Ser Arg Leu Phe Asp Asn Ala Met Leu Arg

1               5                   10                  15

Ala His Arg Leu His Gln Leu Ala Phe Asp Thr Tyr Gln Glu Phe Glu

            20                  25                  30
Glu Ala Tyr Ile Pro Lys Glu Gln Lys Tyr Ser Phe Leu Gln Asn Pro
        35                  40                  45
Gln Thr Ser Leu Cys Phe Ser Glu Ser Ile Pro Thr Pro Ser Asn Arg
    50                  55                  60
Glu Glu Thr Gln Gln Lys Ser Asn Leu Glu Leu Leu Arg Ile Ser Leu
65                  70                  75                  80
Leu Leu Ile Gln Ser Trp Leu Glu Pro Val Gln Phe Leu Arg Ser Val
                85                  90                  95
Phe Ala Asn Ser Leu Val Tyr Gly Ala Ser Asp Ser Asn Val Tyr Asp
            100                 105                 110
Leu Leu Lys Asp Leu Glu Glu Gly Ile Gln Thr Leu Met Gly Arg Leu
        115                 120                 125
Glu Asp Gly Ser Pro Arg Thr Gly Gln Ile Phe Lys Gln Thr Tyr Ser
    130                 135                 140
Lys Phe Asp Thr Asn Ser His Asn Asp Asp Ala Leu Leu Lys Asn Tyr
145                 150                 155                 160
Gly Leu Leu Tyr Cys Phe Arg Lys Asp Met Asp Lys Val Glu Thr Phe
                165                 170                 175
Leu Arg Ile Val Gln Cys Arg Ser Val Glu Gly Ser Cys Gly Phe Phe
            180                 185                 190
Ser Gly Ser Glu Ala Thr Ala Ala Ile Leu Ser Arg Ala Pro Trp Ser
        195                 200                 205
Leu Gln Ser Val Asn Pro Gly Leu Lys Thr Asn Ser Ser Lys Glu Pro
    210                 215                 220
Lys Phe Thr Lys Cys Arg Ser Pro Glu Arg Glu Thr Phe Ser Cys His
225                 230                 235                 240
Trp Thr Asp Glu Val His His Gly Thr Lys Asn Leu Gly Pro Ile Gln
                245                 250                 255
Leu Phe Tyr Thr Arg Arg Asn Thr Gln Glu Trp Thr Gln Glu Trp Lys
            260                 265                 270

Glu Cys Pro Asp Tyr Val Ser Ala Gly Glu Asn Ser Cys Tyr Phe Asn
        275                 280                 285
Ser Ser Phe Thr Ser Ile Trp Ile Pro Tyr Cys Ile Lys Leu Thr Ser
    290                 295                 300
Asn Gly Gly Thr Val Asp Glu Lys Cys Phe Ser Val Asp Glu Ile Val
305                 310                 315                 320
Gln Pro Asp Pro Pro Ile Ala Leu Asn Trp Thr Leu Leu Asn Val Ser
                325                 330                 335
Leu Thr Gly Ile His Ala Asp Ile Gln Val Arg Trp Glu Ala Pro Arg
            340                 345                 350
Asn Ala Asp Ile Gln Lys Gly Trp Met Val Leu Glu Tyr Glu Leu Gln
        355                 360                 365
Tyr Lys Glu Val Asn Glu Thr Lys Trp Lys Met Met Asp Pro Ile Leu
    370                 375                 380
Thr Thr Ser Val Pro Val Tyr Ser Leu Lys Val Asp Lys Glu Tyr Glu
385                 390                 395                 400
Val Arg Val Arg Ser Lys Gln Arg Asn Ser Gly Asn Tyr Gly Glu Phe
                405                 410                 415
Ser Glu Val Leu Tyr Val Thr Leu Pro Gln Met Ser Gln
            420                 425

## Description

The invention relates to a pharmaceutical composition for a long acting growth hormone fusion protein (LAGH), and more particularly a pharmaceutical composition for parenteral administration.

Growth hormone (GH) is a peptide hormone that stimulates growth, cell reproduction and regeneration in humans and other animals. Growth hormone is a 191-amino acid, single-chain polypeptide that is synthesized, stored, and secreted by the somatotroph cells within the lateral wings of the anterior pituitary gland.

GH acts through its receptor, the growth hormone receptor or GHR. The GHR is a transmembrane receptor. Binding of growth hormone to the receptor leads to receptor dimerization and the activation of an intra- and intercellular signal transduction pathway leading to growth. The extracellular domain of the growth hormone receptor (GHR_{ECD}) circulates as a binding protein, which naturally prolongs the biological half-life of growth hormone.

GH is used as a drug *i.a*. for the treatment of child or adult GH deficiency as well as disorders that are unrelated to GH deficiency, such as Turner syndrome, chronic renal failure, Prader-Willi syndrome, intrauterine growth retardation, and severe idiopathic short stature.

As other peptide hormones, GH has a short plasma half-life and requires frequent administration. GH replacement therapy involves daily injections. The extracellular domain of the growth hormone receptor circulates as a binding protein, which naturally prolongs the biological half-life of growth hormone.

Ligand-receptor fusion proteins of growth hormone and the extracellular domain of its receptor have been described in WO2003070765 and WO2001096565. Wilkinson et al. (Nature Medicine 13, 1108 - 1113 (2007)) described a genetically engineered ligand-receptor fusion protein that was purified from mammalian cell culture. In rats, this ligand-receptor fusion had a 300-times reduced clearance as compared to native growth hormone, and a single injection promoted growth for 10 days, far exceeding the growth seen after administration of native growth hormone.

Wilkinson et al. also showed that the ligand-receptor fusion forms a reciprocal, head-to-tail dimer that provides a reservoir of inactive hormone similar to the natural reservoir of growth hormone and its binding protein. It was hence shown that a ligand-receptor fusion of growth hormone to its extracellular receptor generates a potent, long-acting agonist with exceptionally slow absorption and elimination.

Such a fusion protein, called "long acting growth hormone" or LAGH herein, has a potential for treatment of disease with less frequent injections than native growth hormone, such a treatement once a week, every two weeks or once a month.

As all drugs, protein drugs need to be formulated into a pharmaceutical composition so as to be stable and suitable for administration to humans and animals.

So far, no formulation for a LAGH has been described. The present invention proposes such a formulation that is stable over at least 1 week at room temperature and is suitable for treatment of diseases.

The object of the present invention is a pharmaceutical liquid composition comprising,
i) a long acting growth hormone fusion protein (LAGH) as active substance ;
ii) a buffer ; and
iii) a salt ;
   wherein the pH of the composition is between 5.0 and 7.0.

Unless otherwise indicated the following definitions are set forth to illustrate and define the meaning and scope of the various terms used to describe the invention herein.

Unless otherwise stated, all percentages mentioned in the present invention are weight percentages (w/w).

The term "long acting growth hormone fusion protein" or LAGH, as used herein, refers to a fusion protein comprising at least a growth hormone (GH), or a receptor-binding portion thereof, and the extracellular domain of the growth hormone receptor (GHR_{ECD}), or a portion thereof. The receptor-binding portion of growth hormone and the extracellular domain of the growth hormone receptor (GHR_{ECD}), or a portion thereof are either fused directly, or through a suitable linker.

A "fusion protein", as used herein, refers to a fusion of two or more amino acid sequences that are not naturally linked together. The fusion of the amino acid sequences can either be made by chemicaly linking *ex vivo* synthezised amino acid sequences. In an embodiment, the amino acid sequences are an in-frame translational fusion, i.e. correspond to a recombinant molecule expressed from a nucleic acid sequence in a prokaryotic or eukaryotic expression system.

The term "amino acid sequence", as used herein, relates to a sequence of two or more amino acids, such as in a peptide, polypeptide or protein.

The term "long acting growth hormone" or LAGH, as used herein, refers to a fusion protein comprising at least a receptor-binding portion of growth hormone (GH) and the extracellular domain of the growth hormone receptor (GHR_{ECD}), or a portion thereof. The receptor-binding portion of growth hormone and the extracellular domain of the growth hormone receptor (GHR_{ECD}), or a portion thereof are either fused directly, or through a suitable linker.

The term "growth hormone", as used herein, relates to human growth hormone or any growth hormone ortholog such as a growth hormone from an other animal species such as rat, mouse, pig or rabbit.

The term "a receptor-binding portion of growth hormone", as used herein, relates to a portion of growth hormone that contains growth hormone site 1 and/or growth hormone site 2, as defined e.g. in Wells, PNAS 93, pp. 1-6, 1996. The term "a receptor-binding portion of growth hormone" also refers to a human growth hormone that contains at least the following amino acid residues of human growth hormone: 41, 45, 61, 64, 172, 175, 176 and 178, or the homologous residues of a growth hormone of another species.

The term "extracellular domain of a growth hormone receptor (GHR_{ECD}), or a portion thereof", as used herein relates to a protein that either contains residues 1 to 246 of the human growth hormone receptor, or a portion thereof, or the homologous region of the growth hormone receptor of another species. A portion of the extracellular domain of the growth hormone receptor can e.g. contain residues 1 to 238 or residues, or residues 1 to 123 and/or residues 128 to 238 of the human growth hormone receptor or the homologous region(s) of the growth hormone receptor of another species. The term "a receptor-binding portion of growth hormone" also refers to a human growth hormone receptor that contains at least the following amino acid residues of human growth hormone: 43, 44, 103, 104, 105, 106, 164, 165 and 169, or the homologous residues of a growth hormone receptor of another species.

The term "growth hormone receptor agonist", as used herein, refers to a compound that activates the growth hormone receptor when measured e.g. in a transcription assay that is described by Ross, RJ. M. et al. Journal of Clinical Endocrinology & Metabolism. 86, 17161723 (2001). The activation can e.g. be 90% or 100% or 110% of the activation (induction of reporter gene expression) exhibited by native growth hormone.

A "linker", as used herein, can be any suitable chemical entity and preferably a peptide, which comprises 5 to 30 amino acid residues. In an embodiment, the linker comprises 10 to 20 amino acid residues. In another embodiment, the linker comprises at least one copy of the peptide: Gly Gly Gly Gly Ser (hereinafter referred to as "Gly₄Ser").

In embodiments of the invention, the linker comprises two, three, four or five copies of the Gly₄Ser linker.

A "nucleic acid molecule that hybridizes under stringent hybridization conditions" is defined as follows:
Hybridization of a nucleic acid molecule occurs when two complementary nucleic acid molecules undergo an amount of hydrogen bonding to each other. The stringency of hybridization can vary according to the environmental conditions surrounding the nucleic acids, the nature of the hybridization method, and the composition and length of the nucleic acid molecules used. Calculations regarding hybridization conditions required for attaining particular degrees of stringency are discussed in Sambrook et al., Molecular Cloning: A Laboratory Manual (Cold Spring Harbor Laboratory Press, Cold Spring Harbor, NY, 2001); and Tijssen, Laboratory Techniques in Biochemistry and Molecular Biology - Hybridization with Nucleic Acid Probes Part I, Chapter 2 (Elsevier, New York, 1993). The Tm is the temperature at which 50% of a given strand of a nucleic acid molecule is hybridized to its complementary strand. The following is an exemplary set of hybridization conditions and is not limiting:
   - Very High Stringency (allows sequences that share at least 90% identity to hybridize):
      Hybridization: 5x SSC at 65°C for 16 hours;
      Wash twice: 2x SSC at room temperature (RT) for 15 minutes each;
      Wash twice: 0.5x SSC at 65°C for 20 minutes each.

   - High Stringency (allows sequences that share at least 80% identity to hybridize):
      Hybridization: 5x-6x SSC at 65°C-70°C for 16-20 hours
      Wash twice: 2x SSC at RT for 5-20 minutes each
      Wash twice: 1 x SSC at 55°C-70°C for 30 minutes each

   - Low Stringency (allows sequences that share at least 50% identity to hybridize)
      Hybridization: 6x SSC at RT to 55°C for 16-20 hours
      Wash at least twice: 2x-3x SSC at RT to 55°C for 20-30 minutes each.
      The term "percent identity", as used herein, is measured as follows:
         To determine the percent identity of two polypeptides/proteins, the amino acid sequences are aligned for optimal comparison purposes (e.g., gaps can be introduced in the sequence of a first amino acid sequence for optimal alignment with a second amino acid sequence). The amino acid residues at corresponding amino acid positions are then compared. When a position in the first sequence is occupied by the same amino acid residue as the corresponding position in the second sequence, then the molecules are identical at that position. The percent identity between the two sequences is a function of the number of identical positions shared by the sequences (i.e., % identity=# of identical positions/total # of positions (e.g., overlapping positions)×100).

The determination of percent identity between two sequences can be accomplished using a mathematical algorithm. A preferred, non-limiting example of a mathematical algorithm utilized for the comparison of two sequences is the algorithm of Karlin and Altschul (1990) Proc. Natl. Acad. Sci. USA 87:2264-2268, modified as in Karlin and Altschul (1993) Proc. Natl. Acad. Sci. USA 90:5873-5877. Such an algorithm is incorporated into the NBLAST and XBLAST programs of Altschul, et al. (1990) J. Mol. Biol. 215:403-410. BLAST nucleotide searches can be performed with the NBLAST program, score=100, wordlength=12 to obtain nucleotide sequences homologous to a nucleic acid molecules of the invention. BLAST variant searches can be performed with the XBLAST program, score=50, wordlength=3 to obtain amino acid sequences homologous to a variant molecules of the invention. To obtain gapped alignments for comparison purposes, Gapped BLAST can be utilized as described in Altschul et al. (1997) Nucleic Acids Res. 25:3389-3402. Alternatively, PSI-Blast can be used to perform an iterated search which detects distant relationships between molecules. When utilizing BLAST, Gapped BLAST, and PSI-Blast programs, the default parameters of the respective programs (e.g., XBLAST and NBLAST) can be used. Another preferred, non-limiting example of a mathematical algorithm utilized for the comparison of sequences is the algorithm of Myers and Miller, (1988) CABIOS 4:11-17. Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used. Yet another useful algorithm for identifying regions of local sequence similarity and alignment is the FASTA algorithm as described in Pearson and Lipman (1988) Proc. Natl. Acad. Sci. USA 85:2444-2448. When using the FASTA algorithm for comparing nucleotide or amino acid sequences, a PAM120 weight residue table can, for example, be used with a k-tuple value of 2.

The percent identity between two sequences can be determined using techniques similar to those described above, with or without allowing gaps. In calculating percent identity, only exact matches are counted.

The term "buffer" as used herein refers to a solution containing an ionic compound that resists changes in its pH. A buffer generally consists in either a weak acid or its salts or a weak base and its salts, which is resistant to changes in pH.

The term "surfactant" as used herein refers to a compound or excipient with surface active properties, used mainly in the present formulations to improve the aqueous solubility of the active ingredient, limit adsorption at solid surfaces and interfaces, help to protect the active substance against degradation and limit *in vitro* active ingredient precipitation.

The term "anti-oxidant" means a pharmaceutically acceptable compound having antioxidant properties in order to prevent oxidative degradation of the active substance and prevent oxidative degradation of the excipients.

In a preferred embodiment, the LAGH comprises a receptor-binding portion of a growth hormone. In a preferred embodiment, the growth hormone is human growth hormone.

In another preferred embodiment, the LAGH comprises an extracellular domain of a growth hormone receptor (GHR_{ECD}), or a portion thereof. In a preferred embodiment, the growth hormone receptor is human growth hormone receptor.

In a more preferred embodiment, the LAGH comprises an amino acid sequence selected from an amino acid encoded by a nucleic acid molecule comprising a nucleic acid sequence selected from :
i) a nucleic acid sequence as represented in SEQ ID NO:1;
ii) a nucleic acid sequence as represented in SEQ ID NO:2;
iii) a nucleic acid sequence as represented in SEQ ID NO:3;
iv) a nucleic acid sequence as represented in SEQ ID NO:4 ; or
v) a nucleic acid molecule comprising a nucleic sequence that hybridizes under stringent hybridization conditions to the complement of SEQ ID NO:1, SEQ ID NO: 2, SEQ ID NO: 3 or SEQ ID NO: 4,
and which encodes a polypeptide that has growth hormone receptor agonist activity.

In another more preferred embodiment, the LAGH comprises an amino acid sequence selected from:
i) an amino acid sequence as represented in SEQ ID NO:5;
ii) an amino acid sequence as represented in SEQ ID NO:6;
iii) an amino acid sequence as represented in SEQ ID NO:7;
iv) an amino acid sequence as represented in SEO ID NO:8;
v) an amino acid sequence as represented in SEQ ID NO:9;
vi) an amino acid sequence as represented in SEO ID NO: 10;
vii) an amino acid sequence as represented in SEQ ID NO:11;
viii) an amino acid sequence as represented in SEQ ID NO: 12;
or a sequence having 90, 91, 92, 93, 94, 95, 96, 97, 98, 99 or 99.5% identity of one of the sequences of SEQ ID NOs 5, 6, 7, 8, 9, 10, 11 or 12,
and wherein said polypeptide has growth hormone receptor agonist activity.

The pH of the composition according to the present invention is between 5.0 and 7.0, preferably between 5.5 and 6.5, and more preferably is 6 ± 0.2.

The composition according to the present invention comprises a buffer. The buffer may be selected for instance from succinic acid or its salts such as sodium succinate, histidine or its salts such as histidine monohydrochloride monohydrate, citric acid or its salts such as sodium citrate, phosphate or its salts such as disodium hydrogen phosphate dodecahydrate, sodium dihydrogen phosphate dihydrate or potassium dihydrogen phosphate, glycine or its derivatives such as glycylglycine, acetic acid or its salts such as sodium acetate, and imidazole.

In a preferred embodiment, the buffer is selected from phosphate or its salts, histidine or its salts, succinic acid or its salts, and citric acid or its salts. In a more preferred embodiment, the buffer is selected from citric acid or its salts, and preferably the buffer is sodium citrate.

In a preferred embodiment, the concentration of the buffer in the composition of the present invention is from 0.1 to 50 mM, preferably between 10 and 50 mM, and more preferably from 10 to 25 mM. Preferably, the buffer is selected from phosphate or its salts, histidine or its salts, succinic acid or its salts, and citric acid or its salts, with a concentration of the buffer from 0.1 to 50 mM. In a more preferred embodiment, the buffer in the composition is sodium citrate at a concentration between 10 and 25 mM.

The composition according to the present invention also comprises a salt. The salt may be selected from anionic salts such as for instance acetate salts or chloride salts, or from cationic salts such as ammonium salts, potassium salts, sodium salts, magnesium salts or calcium salts. The anionic or cationic salts may be monovalent or divalent. Ammonium salts such as ammonium sulphate and sodium salts such as sodium chloride are monovalent cationic salts whereas magnesium salts such as magnesium chloride or calcium salts such as calcium chloride are divalent cationic salts.

In a preferred embodiment, the salt of the composition according to the present invention is a cationic salt, and preferably a cationic mono- or di-valent salt. In a more preferred embodiment, the salt is selected from ammonium salts, sodium salts, magnesium salts or calcium salts

In a more preferred embodiment, the salt is selected from ammonium and sodium salts, and more preferably from ammonium sulphate and sodium chloride.

In a preferred embodiment, the concentration of the salt in the composition is between 5 to 200 mM, and preferably between 10 to 150 mM. In a more preferred embodiment, the salt is selected from ammonium or sodium salts and its concentration in the composition is between 5 to 200 mM, and preferably between 10 to 150 mM.

In a preferred embodiment, the ratio LAGH / salt (w/w) in the composition according to the invention is between 2:1 and 1:20 and preferably between 1:1 and 1:15.

The composition of the present invention comprises a LAGH, a buffer and a salt. The composition may also comprise further excipients. Such excipients may be any excipients usually used in protein formulation. The further excipients are preferably selected from sugars, amino-acids, polyols, cyclodextrins, surfactants, polymers and antioxidants. The excipient may be an excipient alone or a mixture of excipients. The sugars may be selected from mannitol, sucrose or trehalose. The amino-acids may be selected from L-arginine, L-histidine, L-lysine, glycine or L-aspartic acid. The polyols may be selected from glycerol. The cyclodextrins may be selected from hydroxypropyl beta cyclodextrin. The surfactants may be selected from Polysorbate 20, Polysorbate 80, Poloxamer 188, Solutol HS15 or sucrose-based esters such as sucrose monooleate or sucrose monolaurate. The polymers may be selected from polyethylene glycol (PEG 400, PEG 3350) or povidone K17. The antioxidants may be selected from methionine or ascorbic acid, or antioxidant polymer such as α-tocopherol-PEG-succinate. The excipient may be an excipient alone or a mixture of excipients.

In a preferred embodiment, the composition according to the present invention comprises at least a further excipient. In a more preferred embodiment, the further excipient is selected from sugars and/or surfactants. In a more preferred embodiment, the further excipient is selected from sugars. In a more preferred embodiment, the composition according to the present invention comprises a sugar which is selected from mannitol, sucrose and trehalose. In a more preferred embodiment, the further excipient is selected from sugars and surfactants. In a more preferred embodiment, the composition according to the present invention comprises sugars and surfactants selected from mannitol, sucrose, trehalose, Polysorbate 20, Polysorbate 80, Poloxamer 188, Solutol HS15 and sucrose-based esters.

The composition of the present invention may be prepared by admixing successively, the buffer, the salt, optionally further excipients, and then the liquid LAGH bulk. The pH of the final solution is then measured. Further excipients may also be added to the solution in order to obtain the final formulation.

A pharmaceutical composition according to the present invention is administered by parenteral route. In a preferred embodiment, the composition of the present invention is administered by subcutaneous route.

A pharmaceutical composition according to the present invention may be useful in the treatment of GH deficiency including Turners Syndrome, Prader Willi Syndrome, Interuterine growth retardation, idiopathic short stature, renal failure, catabolic states for example during chemotherapy treatment and in the treatment of AIDS; osteoporosis; diabetes mellitus; cancer; obesity; insulin resistance; hyperlipidaemia; hypertension; anaemia; autoimmune and infectious disease; inflammatory disorders including rheumatoid arthritis (IL-6 chimera).

The composition according to the present invention may be useful as a medicament, in particular as a medicament to treat GH deficiency.

In a preferred embodiment, the composition according to the present invention is used as a medicament. In a more preferred embodiment, the composition according to the present invention is used as a medicament intended to treat GH deficiency.

The following examples are presented to illustrate the above procedures and should not be considered as limiting the scope of the invention.

### Experimental part

### Example 1 : Study of buffer type and molarity value

The concentration of the LAGH bulk is initially 5.4 mg/ml in a PBS buffer with the following composition : 11.9 mM phosphate, 137 mM NaCl and 2.7 mM KCI at pH 7.4.

This initial bulk solution is diluted by 1/10^{th} in various buffers at pH 6. Then the salt and optional further excipients can be added.

The physical integrity of the fusion LAGH protein is characterized using SE-HPLC. The method measures the variation of LAGH monomer purity, content in dimers, oligomers and higher molecular weight compounds. It also determines the content of free GH, as an impurity resulting from clipping of the LAGH fusion protein. The SE-HPLC method is as follows:

| | |
|---|---|
| Column | Superdex 200, GE Healthcare, 10 mm x 300 mm GL, Cat # 17-5175-01 |
| Column temperature | 25° C |
| Injector temperature | 4° C |
| Mobile phase | PBS buffer: 11.9 mM phosphate, 137 mM NaCl, 2.7 mM KCI, pH 7.4 |
| Flow | 1 mUmin - isocratic |
| Injected volume | 100 µL |
| Working concentration | 1 mg /mL |
| Detection | UV at 214 nm |
| Analysis duration | 30 minutes |
| Solvent of dilution | PBS buffer: 11.9mM phosphate, 137 mM NaCl, 2.7 mM KCI, pH 7.4 |
| Needle rinsing | Water / Ethanol (80/20) |

The content and total chemical impurities (oxidation, proteolysis, deamidation) are measured by RP-HPLC at T0 and after one week of storage at room temperature or 25 °C.

The RP-HPLC method is as follows :

| | | | |
|---|---|---|---|
| Column | Bridge BEH 300 C4 waters 2.1 x 250mm 3.5 µm + Symmetry 300 C4 waters guard column 2.1 x 250mm 3.5 µm | | |
| Column temperature | 60° C | | |
| Injector temperature | 4° C | | |
| Mobile phase | Buffer A : Milli Q water / 0.1%TFA | | |
| | Buffer B : acetonitrile / 0.1 %TFA | | |
| Analysis duration | 15 minutes | | |
| Flow | 0.5 mUmin - gradient | | |

| | Time | %A | %B |
|---|---|---|---|
| | 0 | 80 | 20 |
| | 1 | 80 | 20 |
| | 3 | 40 | 60 |
| gradient | 10 | 30 | 70 |
| | 10.5 | 0 | 100 |
| | 12.5 | 0 | 100 |
| | 13 | 80 | 20 |
| | 15 | 80 | 20 |
| Injected volume | 10 µL | | |
| Working concentration | 1 mg /mL | | |
| Detection | UV at 214 nm | | |
| Analysis duration | 30 minutes | | |
| Solvent of dilution | PBS buffer: 11.9mM phosphate, 137 mM NaCl, 2.7 mM KCI, pH 7.4 | | |
| Needle rinsing | Water / Ethanol (80/20) | | |

The SE-HPLC and RP-HPLC results are presented in Table 1.

For all formulations, the T0 values were :
- in SEC-HPLC:
   - Initial monomer content: 66.0 ± 1.8 %,
   - Initial dimer content: 24.6 ± 1.5 %,
   - Initial oligomers content: 8.4 ± 0.4 %,
   - Initial free GH content: 1.0 ± 0.1 %.
- in RP-HPLC:
   - Initial LRGH content: 0.54 ± 0.02 mg/mL,
   - Initial impurities level: 11.3 ± 0.5 %.

**Table 1- HPLC results for various buffer types and molarity values.**

| Buffers | Molarity (mM) | pH variation after 1 week at RT | Physical integrity after 1 week at RT (SE-HPLC) | | | | Chemical stability after 1 week at RT (RP-HPLC) | | |
|---|---|---|---|---|---|---|---|---|---|
| | | | Difference of content vs. T0 (%) | | | | Content (mg/ml) | Total impurities (%) | Difference vs. T0 (%) |
| | | | Monomer (%) | Dimer (%) | Oligomers and other (%) | Free GH (%) | | | |
| Phosphate | 10 | -0.08 | + 12 | -10 | -2 | + 0.6 | 0.53 | 14.8 | + 3.9 |
| | 20 | 0.07 | + 9 | -7 | -2 | + 0.5 | 0.54 | 14.1 | + 1.9 |
| | 30 | - 0.06 | + 10 | -9 | -1 | + 0.5 | 0.53 | 14.1 | + 3.0 |
| | 40 | - 0.05 | + 10 | -10 | -1 | + 0.2 | 0.53 | 12.9 | + 1.7 |
| Histidine | 10 | + 0.01 | + 12 | -11 | -2 | + 0.8 | 0.52 | 16.1 | + 5.0 |
| | 20 | 0.00 | + 8 | -7 | -1 | + 0.3 | 0.55 | 14.1 | + 1.8 |
| | 30 | - 0.01 | + 10 | -9 | -2 | + 0.7 | 0.54 | 15.1 | + 4.0 |
| | 40 | - 0.03 | + 10 | -9 | -2 | + 0.6 | 0.53 | 14.8 | + 3.8 |
| | 50 | - 0.02 | + 10 | -9 | -2 | + 0.4 | 0.53 | 14.7 | + 3.6 |
| Citrate | 10 | - 0.07 | + 12 | -11 | -1 | + 0.2 | 0.53 | 14.1 | + 3.2 |
| | 20 | - 0.01 | + 11 | -9 | -2 | + 0.7 | 0.54 | 15.0 | + 3.0 |
| | 30 | - 0.03 | + 11 | -9 | -1 | + 0.3 | 0.52 | 13.5 | + 2.5 |
| | 40 | - 0.06 | + 11 | -10 | -2 | + 0.3 | 0.54 | 13.6 | + 2.6 |
| | 50 | + 0.14 | + 11 | -9 | -2 | + 0.2 | 0.53 | 13.6 | + 2.6 |

### Example 2: Stability study at 25°C for various salt and sugar concentrations

Samples are prepared in different buffers according to the process described in example 1 with 20 mM citrate and phosphate buffers at pH 6.0.

The content and total chemical impurities are measured by RP-HPLC at T0 and after one week of storage at room temperature or 25°C.

The RP-HPLC method is identical to the one described in Example 1.

| Column | Bridge BEH 300 C4 waters 2.1 x 250mm 3.5 µm + Symmetry 300 C4 waters guard column 2.1x 250mm 3.5 µm | | |
|---|---|---|---|
| Column temperature | 60° C | | |
| Injector temperature | 4° C | | |
| Mobile phase | Buffer A : Milli Q water / 0.1%TFA | | |
| | Buffer B : acetonitrile / 0.1 %TFA | | |
| Analysis duration | 15 minutes | | |
| Flow | 0.5 mL/min - gradient | | |

| | Time | %A | %B |
|---|---|---|---|
| | 0 | 80 | 20 |
| | 1 | 80 | 20 |
| | 3 | 50 | 50 |
| Gradient | 10 | 40 | 60 |
| | 10.5 | 0 | 100 |
| | 12.5 | 0 | 100 |
| | 13 | 80 | 20 |
| | 15 | 80 | 20 |
| Injected volume | 10 µL | | |
| Working concentration | 1 mg /mL | | |
| Detection | UV at 214 nm | | |
| Solvent of dilution | PBS buffer: 11.9mM phosphate, 137 mM NaCl, 2.7 mM KCl, pH 7.4 | | |
| Needle rinsing | Water / Ethanol (80/20) | | |

The RP-HPLC results for different salts and sugar are shown in Tables 2 and 3.

**Table 2**

| | | | | | |
|---|---|---|---|---|---|
| The initial LAGH content is 0.48 ± 0.01 mg/mL. | | | | | |

| Buffers | Salt concentration | pH variation after 1 week | Chemical stability after 1 week at 25°C (RP-HPLC) | | |
|---|---|---|---|---|---|
| | | | Content (mg/ml) | Initial (T0) Impurity level (%) | Impurity level variatio vs. T0 (%) |
| phosphate | NaCl 0.08 % | + 0.11 | 0.43 | 11.0 | + 10.0 |
| | NaCl 0.08% (NH₄)₂SO₄ 1.3% | - 0.06 | 0.39 | 11.2 | + 13.8 |
| citrate | NaCl 0.08% | + 0.32 | 0.45 | 11.0 | + 5.2 |
| | NaCl 0.76% | - 0.05 | 0.44 | 9.9 | + 4.5 |
| | NaCl 0.08% (NH₄)₂SO₄ 1.3% | + 0.05 | 0.45 | 10.0 | + 4.2 |

**Table 3**

| | | | | | |
|---|---|---|---|---|---|
| The initial LAGH content is 0.48 ± 0.01 mg/ml. For all formulations, the NaCl salt concentration is 13.7 mM (0.08%). | | | | | |

| Buffers | Sugar concentration | pH variation after 1 week | Chemical stability after 1 week at 25°C | | |
|---|---|---|---|---|---|
| | | | Content (mg/ml) | Initial (T0) impurity level (%) | Impurity level variation vs. T0 (%) |
| phosphate | / | + 0.11 | 0.43 | 11.0 | + 10.0 |
| | mannitol 4% | + 0.14 | 0.37 | 10.0 | + 18.2 |
| citrate | / | + 0.32 | 0.45 | 11.0 | + 5.2 |
| | sucrose 6.5 % | + 0.07 | 0.42 | 9.8 | + 13.1 |
| | trehalose 7.6% | + 0.18 | 0.45 | 10.8 | + 4.7 |

## Claims

1. A pharmaceutical liquid composition comprising,
i) a long acting growth hormone fusion protein (LAGH) as active substance
ii) a buffer ; and
iii) a salt ;
wherein the pH of the composition is between 5.0 and 7.0.

2. The composition according to any of the preceding claims wherein the pH of the composition is between 5.5 and 6.5, and preferably is 6 ± 0.2.

3. The composition according to any of the preceding claims, wherein the buffer is selected from phosphate or its salts, histidine or its salts, succinic acid or its salts and citric acid or its salts.

4. The composition according to any of the preceding claims, wherein the concentration of the buffer in the composition is from 0.1 to 50 mM, and preferably from 10 to 50 mM.

5. The composition according to claim 3 wherein the buffer is sodium citrate.

6. The composition according to claim 5 wherein the buffer is citrate at a concentration in the composition between 10 and 25 mM.

7. The composition according to any of preceding claims wherein the salt is a cationic salt.

8. The composition according to claim 7 wherein the salt is selected from ammonium and sodium salts, and preferably from ammonium phosphate and sodium chloride.

9. The composition according to claim 7 wherein the concentration of the salt in the composition is between 5 to 200 mM, and preferably between 10 to 150 mM.

10. The composition according to any of preceding claims wherein the ratio LAGH / salt (w/w) is between 2:1 and 1:20, and preferably between 1:1 and 1:15.

11. The composition according to any of preceding claims comprising further excipients.

12. The composition according to claim 11 comprising further excipients selected from sugars.

13. The composition according to claim 12 comprising the sugar is selected from mannitol, sucrose and trehalose.

14. The composition according to claim 11 comprising further excipients selected from sugars and surfactants.

15. The composition according to any of preceding claims, for use as medicament.
